# EUROPEAN PATENT SPECIFICATION

(11) **EP 1 106 150 B1**
(45) Date of publication and mention of the grant of the patent: **11.01.2006**
(21) Application number: 00310574.9
(22) Date of filing: 29.11.2000
(51) Int. Cl.: A61F 13/15

(54) **Disposable diaper**
Wegwerfwindel
Couche-culotte jetable

(30) Priority: 30.11.1999 JP 34024899
(43) Date of publication of application: 13.06.2001
(73) Proprietor: UNI-CHARM CORPORATION, Shikokuchuo-shi, Ehime-ken (JP)
(72) Inventor: Sayama, Yashushi, c/o Technical Center, Mitoyo-gun, Kagawa-ken 769-1602 (JP); Minato, Hironao, c/o Technical Center, Mitoyo-gun, Kagawa-ken 769-1602 (JP)
(74) Representative: Parry, Christopher Stephen

(56) References cited:
- EP-A- 0 826 350
- EP-A- 1 057 463
- WO-A-94/18927
- WO-A-99/20215
- US-A- 5 672 164
- US-A- 5 904 675

## Description

This invention relates to a disposable diaper for absorbing and containing excretion.

U.S. Patent No. 4, 695, 278 discloses a disposable diaper provided along transversely opposite side edge portions with a pair of gasketing cuffs and a pair of barrier cuffs. The gasketing cuffs flaps are formed by placing the portion of a topsheet covering a liquid-absorbent core and extending laterally beyond transversely opposite side edges of the core upon the portion of a backsheet extending laterally beyond the side edges of the core. The barrier cuffs are formed by bonding sheets provided separately of the topsheet to the top surface of the topsheet or by the portion of the topsheet partially projecting upward on the respective gasketing cuffs so as to describe inverted U-shapes, respectively. Each of the gasketing cuffs is provided along its outer side edge portion with a longitudinally extending elastic member secured under tension thereto. Each of the barrier cuffs has a proximal side edge portion bonded to the associated gasket cuff and a distal side edge transversely opposed to the proximal side edge and provided with a longitudinally extending elastic member secured under tension thereto.

In the diaper of prior art, the distal side edge portion of the barrier cuff is biased under a contractile force of the elastic member to rise on the top surface of the diaper and thereby to function as a barrier. Contraction of the elastic member of the gasket cuff causes its outer side edge portion to swing around the associated side edge of the core inwardly of the diaper from its initial horizontal position toward the associated barrier cuff rising on the top surface of the diaper. Such tendency raises a problem when the diaper is put on a wearer's body since the gasket cuff which should directly cover the wearer's thighs may collapse inwardly of the diaper. Consequently, it is apprehended that the gasketing cuff could not fulfil its desired function and create a feeling of discomfort against a wearer. Furthermore, the gasketing cuff collapsing inwardly of the diaper may obstruct the barrier cuff from properly rising.

US 5,904,675 and WO 99/20215 disclose diapers with elasticated barrier cuffs and elasticated gasket cuffs formed integrally and subsequently bonded to a topsheet of a diaper in a region adjacent an absorbent core of a diaper.

US 5,672,164 discloses a diaper wherein a gasket cuff is formed from an extended portion of a backsheet of the diaper and an additional sheet bonded to the backsheet, wherein said additional sheet extends to form an elasticated barrier cuff.

It is an object of this invention to provide a disposable diaper improve so that the elastically stretchable side flaps can be reliably prevented from collapsing inwardly of the diaper put on the wearer.

According to this invention, there is provided a disposable diaper having a transverse direction extending circumferentially around a wearer's torso and a longitudinal direction orthogonal to said transverse direction, comprising a liquid-pervious topsheet, a liquid-impervious backsheet, a liquid-absorbent core disposed therebetween and a pair of gasketing cuffs lying outside transversely opposite side edges of said core extending in said longitudinal direction and having elastic stretchability in said longitudinal direction, each said gasketing cuff being formed at least partially by said liquid-impervious backsheet extending laterally from said side edges of said core, and at least one separate sheet laminated with said liquid-impervious sheet and provided with said elastic stretchability by securing a leg-hole elastic member extending in the longitudinal direction to said gasketing cuff along a line spaced from the associated side edge of said core; characterised in that said topsheet extends in said transverse direction, at least in a longitudinal middle region of said diaper, into a portion of said gasketing cuff defined between said side edge of said core and said elastic member, the edge of said topsheet defining a boundary dividing the gasketing cuff, at least in a longitudinal middle region of said diaper, into a high stiffness zone and a low stiffness zone so that said high stiffness zone, comprising said topsheet, lies adjacent said core and said low stiffness zone lies adjacent said elastic member, wherein said high stiffness zone, comprising said top sheet, has a transverse dimension of between 10 and 50 mm.

With the disposable diaper according to this invention, the outer side edge portions of the respective gasket cuff swing outward laterally of the diaper when the diaper is put on the wearer and therefore the gasket cuffs can fulfill the function thereof without collapsing inwardly of the diaper. In this way, it is possible to avoid that a feeling of discomfort against the wearer be created and the barrier cuffs might be obstructed from swinging up both due to collapsing of the gasket cuffs.
Fig. 1 is a perspective view showing one embodiment of a partially cutaway disposable diaper according to this invention;
Fig. 2 is a fragmentary sectional view taken along line II - II in Fig. 1;
Fig. 3 is a view similar to Fig. 2 showing the diaper as it is put on a wearer;
Fig. 4 is a view similar to Fig. 2 showing another embodiment of this invention;
Fig. 5 is a view similar to Fig. 2 showing still another embodiment of this invention;
Fig. 6 is a view similar to Fig. 2 showing further another embodiment of this invention; and
Fig. 7 is a view similar to Fig. 2 showing further additional embodiment of this invention.

Details of a disposable diaper according to this invention will be more fully understood from the description given hereunder with reference to the accompanying drawings.

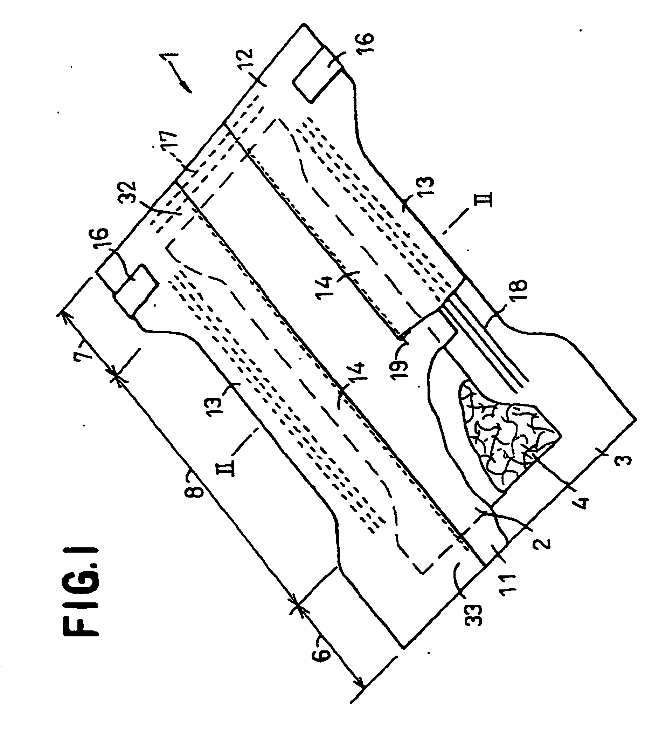

A disposable diaper 1 shown by Fig. 1 in a perspective view as partially cutaway has a transverse direction extending circumferentially around a wearer's torso and a longitudinal direction orthogonal to the transverse direction. The diaper 1 is configured to define, in the longitudinal direction, a front waist region 6, a rear waist region 7 and a crotch region 8 extending between these two waist regions 6, 7. The diaper 1 comprises a liquid-pervious topsheet 2, a liquid-impervious backsheet 3, a liquid-absorbent core 4 disposed between these two sheets 2, 3, a pair of end flaps 11, 12 extending in the transverse direction along longitudinally opposite ends of the core 4 and a pair of gasketing cuffs 13 extending in the longitudinal direction along and outside transversely opposite side edges of the core 4. In addition, the diaper 1 is formed on its inner side with a pair of barrier cuffs 14 extending along the respective gasketing cuffs 13 in the longitudinal direction. A pair of tape fasteners 16 adapted to be anchored on the outer surface of the front waist region 6 are respectively attached to transversely opposite side edge portions of the rear waist region 7. The end flap 12 of the rear waist region 7 is provided with a single or plural waist-hole elastic members 17 extending in the transverse direction and secured under tension to the inner surface of at least one of the top- and backsheets 2, 3 forming this end flap 12. Each of the gasketing cuffs 13 is provided with a single or parallel plural leg-hole elastic members 18 extending in the longitudinal direction and each of the barrier cuffs 14 is provided with a single crotch region elastic member 19 extending in the longitudinal direction.

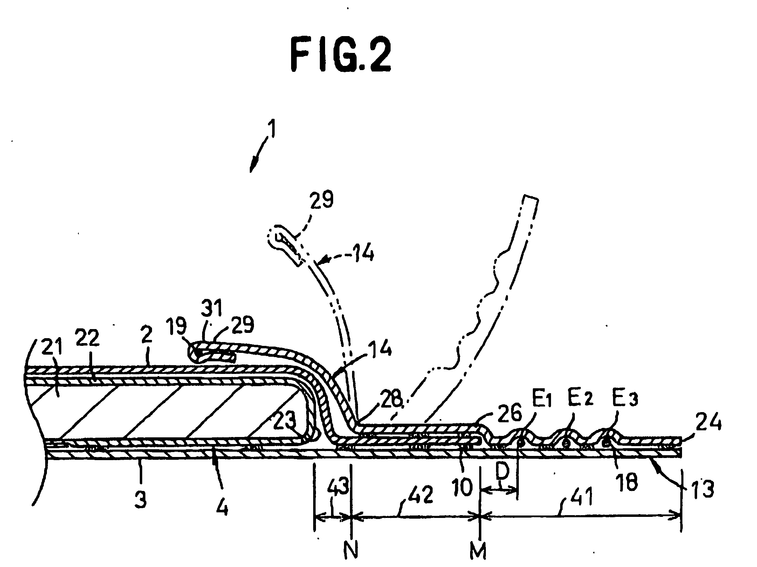

Fig. 2 is a fragmentary sectional view taken along center line II - II bisecting a longitudinal dimension of the diaper 1. The core 4 comprises a wad 21 substantially made of fluff pulp covered with a tissue paper 22. The top- and backsheets 2, 3 extend outward beyond the transversely opposite side edges 23 placed upon and bonded to each other along the respective extensions by means of hot melt adhesive 10. The backsheet 3 extends further outward beyond the topsheet 2 to respective side edges 24 of the horizontal gasketing cuff 13. In each of the gasketing cuffs 13, leg-hole elastic member 18 is secured under tension to the inner surface of the backsheet 3 by means of hot melt adhesive (not shown) and a liquid-impervious sheet 26 bonded to the inner surface of the backsheet 3 and the outer surface of the topsheet 2 by means of adhesive 10 so as to cover the elastic member 18. A portion of this liquid-impervious sheet 26 branched from the gasketing cuff 13 and extending inwardly of the diaper 1 forms the barrier cuff 14. In the case of the embodiment shown, the leg-hole elastic member 18 comprises three elastic elements E₁, E₂, E₃.

The barrier cuff 14 has a proximal side edge 28 along which the barrier cuff 14 is branched from the gasketing cuff 13 and a distal side edge 29 transversely opposed to the proximal side edge 28. The liquid-impervious sheet 26 is folded back and bonded together along the distal side edge 29 to form a sleeve 31 and a crotch region elastic member 19 is secured under tension to the inner surface of the sleeve 31 by means of hot melt adhesive (not shown). The barrier cuff 14 collapses inwardly of the diaper 1 and has its longitudinally opposite end portions 32, 33 (See Fig. 1) are bonded to the outer surface of the topsheet 2.

The gasketing cuff 13 has a laminated construction presenting a sectional shape as shown by Fig. 2 at least along a part of its full length extending in the vicinity of the center line II - II. Referring to Fig. 2, the gasketing cuff 13 comprises, in the vicinity of the center line II - II, a first zone 41 in which the backsheet 3 and the liquid-impervious sheet 26 are placed upon and bonded to each other including the elastic member 18 therebetween and having a relatively low bending stiffness in the transverse direction, a second zone 42 in which the top- and backsheets 2, 3 and the liquid-impervious sheet 26 are placed upon and bonded one to another and having a relatively high bending stiffness in the transverse direction and a third zone 43 defined between the side edge of the core 4 and the proximal side edge 28 of the barrier cuff 14, in which the top- and backsheets 2, 3 are placed upon and bonded to each other. The first zone 41 and the second zone 42 are contiguous to each other along a straight line M while the second zone 42 and the third zone 43 are contiguous to each other along a straight line N. The number of sheets laminated one upon another is different in the respective zones 41, 42, 43. The first, second and third zones 41, 42, 43 respectively have their transverse dimensions p, q, r wherein, preferably, p = 5 - 100 mm, q = 10 - 50 mm and r = 0 - 30 mm. A distance D from the innermost leg-hole elastic member E₁ to the border line M of the second zone 42 is preferably 0.5 - 30 mm, more preferably 1 - 10 mm. The inventors measured stiffness of the first, second and third zones 41, 42, 43 by Clark method prescribed in JIS (Japanese Industrial standards) L-1096 and found that, preferably, the first zone 41 has a stiffness G₁ in a range of 50 - 100 mm, the second zone 42 has a stiffness G₂ in a range of 100 - 250 mm and the stiffness G₂ is at least 30 mm higher than the stiffness G₁. The stiffness G₃ of the third zone 43 is not specified and may be lower or higher than the stiffness G₂. values of the stiffness G₁ - G₃ can be measured using the test pieces prepared to have the same laminated construction as those of the relevant zones.

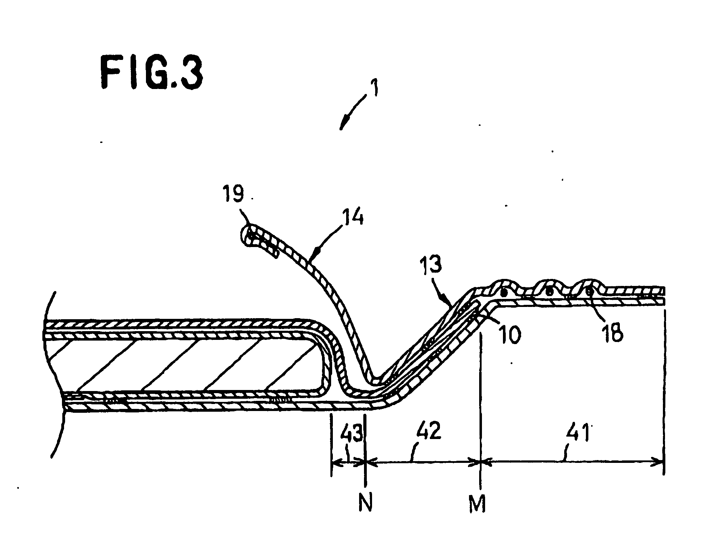

Fig. 3 is a sectional view similar to Fig. 2 showing the diaper 1 immediately before it is put on the wearer. As the diaper 1 constructed as shown in Figs. 1 and 2 is laid on a bed of the wearer immediately before it is put on the wearer, the leg-hole elastic members 18 as well as the crotch region elastic members 19 contract to generate gathers in the respective flaps 13, 14 and, at the same time, the diaper 1 is curved in the longitudinal direction with its inner side being concave. Thereupon the free distal side edge portions 29 of the respective barrier cuffs 14 swing up from the inner surface of the diaper 1 around the respective proximal side edges 28 as indicated by imaginary lines in Fig. 2 and the gasketing cuffs 13 also swing up inwardly of the diaper 1. Taking account of the fact that it is difficult to put the diaper 1 in this curved state on the wearer, the diaper 1 may be slightly tensioned in the longitudinal direction and thereby the diaper 1 is substantially flattened. As a result, the first zone 41 of the gasketing cuffs 13 swings back around an axis defined between the border line M along which the first and second zones 41, 42 are contiguous to each other and the innermost elastic member E₁ to the position at which the first zone 41 extends laterally of the diaper 1. The height of the barrier cuff 14 in its rising state slightly goes down as the diaper 1 is tensioned in the longitudinal direction. The diaper 1 may be put on the wearer with the gasketing cuffs 13 flattened as has been described to prevent the gasketing cuff 13 from collapsing inwardly of the diaper 1. In other words, it is not apprehended that the gasketing cuffs 13 might be held between the core 4 and the wearer. In this way, the side flaps 13 are tightly placed around the wearer's thighs and thereby fulfill their function to reliably prevent leakage of body fluids. It is also avoided that the gasketing cuffs 13 collapsing inwardly of the diaper 1 might create a feeling of discomfort against the wearer and obstruct the barrier cuffs 14 from properly rising on the inner surface of the diaper 1.

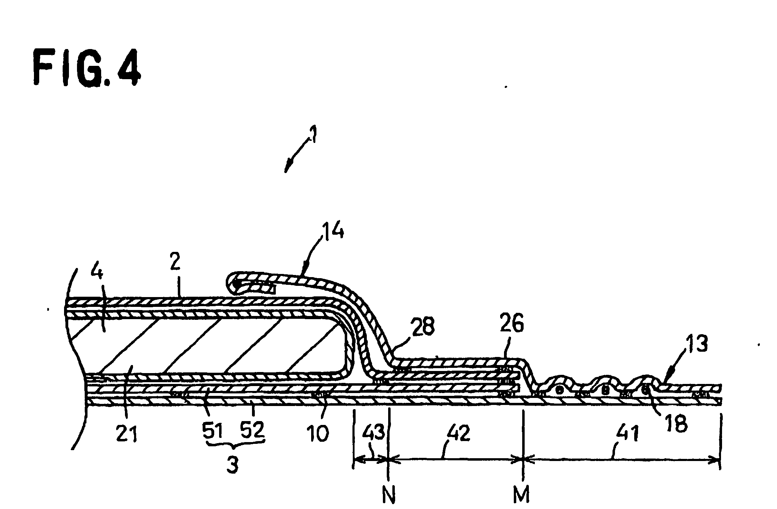

Figs. 4 - 6 are views similar to Fig. 2 showing the other embodiments of this invention. In the case of the diaper 1 shown by Fig. 4, the backsheet 3 is formed by a relatively narrow liquid-impervious plastic film 51 and a relatively large nonwoven fabric 52 bonded to the outer surface of the film 51. The first zone 41 including the leg-hole elastic member 18 comprises two layers, i.e., the nonwoven fabric 52 of the backsheet 3 and the liquid-impervious sheet 26 made of a nonwoven fabric and forming the barrier cuff 14. The second zone 42 comprises four layers, i. e., the topsheet 2 made of a nonwoven fabric, a pair of backsheets 3 and the liquid-impervious sheet 26 and the third zone 43 comprises three layers, i.e., the topsheet 2 and a pair of backsheets 3. The sheets laminated with one another as shown are bonded together by means of hot melt adhesive 10 so that the sheets thus bonded together may present the stiffness increasing in the order of the first zone 41, the third zone 43 and the second zone 42. In the case of this embodiment also, the side flap 13 can swing up and down around the border line along which the first and second zones 41, 42 are contiguous to each other. The first zone 41 can extend laterally of the diaper 1 immediately before the diaper 1 is put on the wearer.

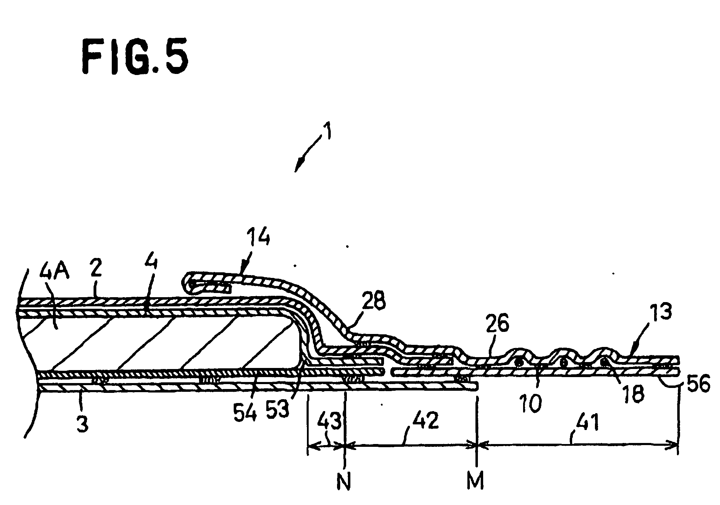

In the case of the diaper 1 shown by Fig. 5, a wad 4A of the core 4 substantially formed by fluff pulp is covered with upper and lower sheets 53, 54 of tissue paper which extend outward beyond the wad 4A and form parts of the gasketing cuffs 13. Of the gasketing cuffs 13, the first zone 41 including the leg-hole elastic member 18 comprises two layers, i.e., a hydrophobic sheet 56 prepared separately of the top- and backsheets 2, 3 and the liquid-impervious sheet 26, on one hand, and the liquid-impervious sheet 26, on the other hand. The second zone 42 partially comprises five layers, i.e., the top- and backsheets 2, 3, the sheets of tissue paper 53, 54 and the liquid-impervious sheet 26 and partially comprises four layers, i.e. , the top- and backsheets 2, 3, the liquid-impervious sheet 26 and the hydrophobic sheet 56. The third zone 43 comprises the top- and backsheets 2, 3 and the sheets of tissue paper 53, 54. In this embodiment, the second zone 42 extending immediately inside the border line M presents the stiffness G₂ at least 30 mm higher than the stiffness G₁ presented by the first zone 41 and the first zone 41 can extend laterally of the diaper 1 as shown in Fig. 2. In this first zone 41, a sheet having a high breathability may be used as stock material for the hydrophobic sheet 56 or for the hydrophobic sheet 56 and the liquid-impervious sheet 26 to improve the breathability desired around the wearer's thighs.

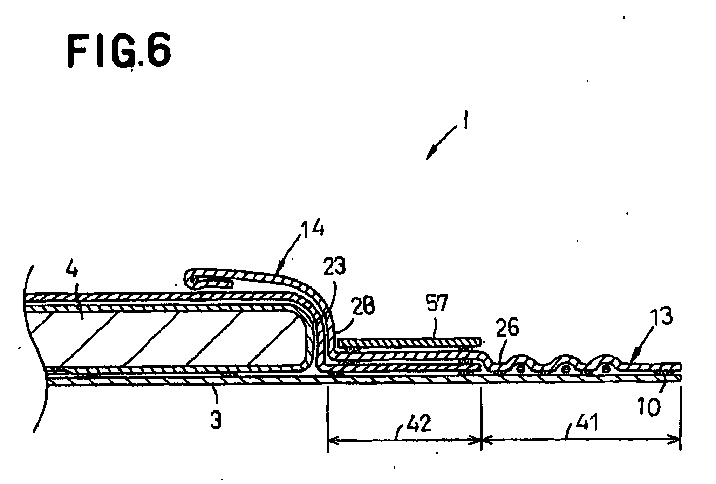

In the case of the diaper 1 shown by Fig. 6, the first zone 41 comprises two layers, i.e., the backsheet 3 and the liquid-impervious sheet 26 and the second zone 42 comprises four layers, i.e., the top- and backsheets 2, 3, the liquid-impervious sheet 26 and an appropriately stiffened sheet 57 prepared separately of these sheets 2, 3, 26. The sheets laminated one with another are bonded together by means of hot melt adhesive 10 or heat-sealing technique and the barrier cuffs 14 lie along the respective side edges 23 of the core 4 and extend inwardly of the diaper 1. In this diaper 1, the dimension r of the third zone 43 is substantially zero, namely, the third zone 43 is practically absent in this diaper 1. The sheet 57 is provided for the purpose of ensuring the second zone 42 to present the stiffness G₂ substantially higher than the stiffness G₁ presented by the first zone 41 and a nonwoven fabric or plastic film meets such purpose. In this diaper 1 also, the first zone 41 can extend outward laterally of the diaper 1 as shown in Fig. 3.

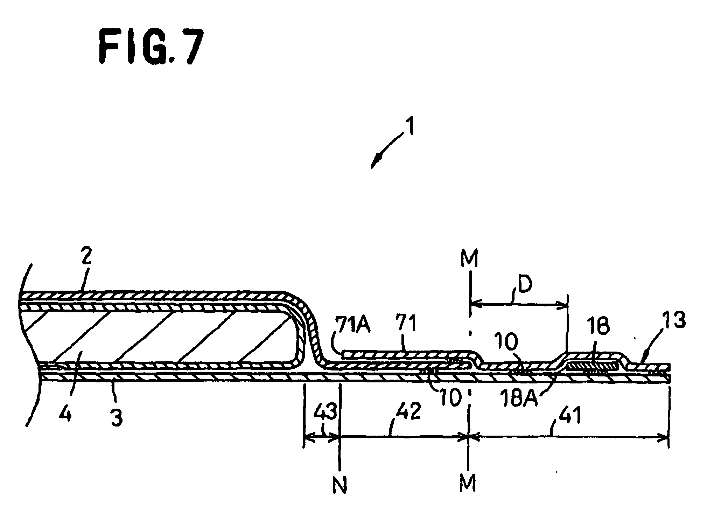

Fig. 7 is also a sectional view similar to Fig. 2 showing further another embodiment of this invention. The diaper 1 of Fig. 7 is distinguished from the diaper 1 of Fig. 2 in that none of the barrier cuffs 14 is provided and a single belt-like elastic member is used as each of the leg-hole elastic members 18. The first zone 41 including the leg-hole elastic member 18 comprises two layers, i.e., the backsheet 3 formed by a liquid-impervious plastic film or nonwoven fabric and an inner sheet 71 preferably formed by liquid-impervious nonwoven fabric, the second zone 42 comprises three layers, i.e., the top- and backsheets 2, 3 and the inner sheet 71 and the third zone 43 comprises two layers, i.e., the top- and backsheets 2, 3. In the first zone 41, the distance D from the inner side edge 18A of the leg-hole elastic member 18 to the border line M along which the first and second zones 41, 42 are contiguous to each other is preferably in a range of 0.5 - 30 mm, more preferably in a range of 1 - 10 mm. While the gasketing cuff 13 swings up as indicated by imaginary lines in Fig. 2 as the diaper 1 is laid on the bed immediately before it is put on the wearer, the diaper 1 may be slightly tensioned in the longitudinal direction to make the gasket cuff 13 swing back in the vicinity of the border line M until the gasketing cuff 13 extends laterally of the diaper 1 as shown in Fig. 3. In order that the gasketing cuff 13 can easily swing back in this manner, the distance D is preferably minimized so that the second zone 42 and the inner side edge 18A of the elastic member 18 may lie in vicinity as close as possible. For the embodiments shown by Figs. 1 - 6 in which the leg-hole elastic member 18 comprises a plurality of elastic elements, the distance D between the innermost elastic element 18 and the second zone 42 is preferably in a range of 0.5-30 mm, more preferably in a range of 1 - 10 mm as has previously been described in reference with Fig. 2. In the diaper 1 of Fig. 7 also, it is not apprehended that the gasketing cuff 13 might collapse inwardly of the diaper 1 and obstruct the associated barrier cuff 14 from swing up. Accordingly, leak of body fluids and creation of a feeling of discomfort against the wearer due to the collapse can be reliably avoided. In this diaper 1, the dimension r of the third zone 43 will become zero when the inner sheet 71 is dimensioned so that its inner side edge 71A may reach the associated side edge 23 of the core 4.

The desired difference between the stiffness G₁ of the first zone 41 and the stiffness G₂ of the second zone 42 can be ensured not only by varying the number of the sheets laminated in the respective zones but also varying thickness as well as material of the sheets laminated one with another.

## Claims

1. A disposable diaper (1) having a transverse direction extending circumferentially around a wearer's torso and a longitudinal direction orthogonal to said transverse direction, comprising a liquid-pervious topsheet (2), a liquid-impervious backsheet (3), a liquid-absorbent core (4) disposed therebetween and a pair of gasketing cuffs (13) lying outside transversely opposite side edges of said core extending in said longitudinal direction and having elastic stretchability in said longitudinal direction, each said gasketing cuff being formed at least partially by said liquid-impervious backsheet (3) extending laterally from said side edges of said core,
and at least one separate sheet (26) laminated with said liquid-impervious sheet and provided with said elastic stretchability by securing a leg-hole elastic member (18) extending in the longitudinal direction to said gasketing cuff (13) along a line spaced from the associated side edge of said core;
**characterised in that** said topsheet (2) extends in said transverse direction, at least in a longitudinal middle region of said diaper, into a portion (42) of said gasketing cuff defined between said side edge (23) of said core (4) and said elastic member, the edge of said topsheet defining a boundary dividing the gasketing cuff, at least in a longitudinal middle region of said diaper, into a high stiffness zone (42) and a low stiffness zone so that said high stiffness zone (41), comprising said topsheet (2), lies adjacent said core and said low stiffness zone lies adjacent said elastic member, wherein said high stiffness zone, comprising said top sheet, has a transverse dimension of between 10 and 50 mm.

2. The diaper according to Claim 1, wherein said diaper (1) is formed on a distal side thereof with a pair of barrier cuffs (14) each formed by a liquid-impervious sheet (26) and having a top side edge having an elastic stretchability in said longitudinal direction and a proximal side edge (28) integral with said top surface of said diaper so that said distal side edge (29) can swing up from said top surface of said diaper as said diaper is longitudinally curved.

3. The diaper according to Claim 2, wherein said proximal side edge (28) lies between the associated side edge (22) of said core (4) and said elastic member (18) at least in said longitudinally middle region of said gasketing cuff and wherein said gasketing cuff is divided between said proximal side edge portion (28) and said elastic member (18) into said high stiffness zone and said low stiffness zone.

4. The diaper according to Claim 1, wherein said gasketing cuff is provided with a single or a plurality of parallel elastic members (18) and a portion of said gasketing cuff extending inside the innermost elastic member is divided into said high stiffness zone and low stiffness zone.

5. The diaper according to Claim 1, wherein said high stiffness zone (42) comprises at least one of said top- and backsheets and a sheet (26) prepared separately of said topsheet and/or backsheet and bonded to said one of the top- and backsheets.

6. The diaper according to Claim 1, wherein a distance from the innermost elastic element of said leg-hole elastic member to said high stiffness zone is in a range of 1 - 10 mm.

7. The diaper according to any preceding claim, wherein said low stiffness zone has a transverse dimension between 5 and 100 mm.

8. The diaper according to any preceding claim, wherein the distance between an edge of said absorbent core and the high stiffness zone is between 0 and 30 mm.

## Patentansprüche

1. Wegwerfwindel (1) mit einer in Umfangsrichtung um den Rumpf des Trägers verlaufenden Querrichtung und einer senkrecht dazu verlaufenden Längsrichtung, mit einer flüssigkeitsdurchlässigen oberen Lage (2), einer flüssigkeitsundurchlässigen unteren Lage (3), einem dazwischen liegenden flüssigkeitsabsorbierenden Kern (4) und einem Paar von Dichtmanschetten (13), die außerhalb von in Querrichtung einander gegenüberliegenden Seitenkanten des Kerns angeordnet sind, in der Längsrichtung verlaufen und in dieser eine elastische Dehnbarkeit aufweisen, wobei jede Dichtmanschette mindestens teilweise von der seitlich der Seitenkanten des Kerns verlaufenden flüssigkeitsundurchlässigen unteren Lage (3) gebildet ist,
und mindestens einer mit der flüssigkeitsundurchlässigen Lage laminierten getrennten Lage (26), die **dadurch** mit der elastischen Dehnbarkeit versehen ist, daß ein in der Längsrichtung verlaufendes elastisches Beinlochteil (18) längs einer in Abstand von der zugehörigen Seitenkante des Kerns verlaufenden Linie an der Dichtmanschette (13) befestigt ist,
**dadurch gekennzeichnet, daß** die obere Lage (2) mindestens in einem Längsmittenbereich der Windel in der Querrichtung in einen zwischen der Seitenkante (23) des Kerns (4) und dem elastischen Teil definierten Abschnitt (42) der Dichtmanschette verläuft, wobei die Kante der oberen Lage eine Grenze definiert, die die Dichtmanschette mindestens in einem Längsmittenbereich der Windel in eine Zone (42) hoher Steifigkeit und eine Zone (41) geringer Steifigkeit unterteilt, so daß die Zone hoher Steifigkeit, die die obere Lage (2) enthält, dem Kern und die Zone geringer Steifigkeit dem elastischen Teil benachbart ist, wobei die die obere Lage enthaltende Zone hoher Steifigkeit eine Querabmessung zwischen 10 und 50 mm hat.

2. Windel nach Anspruch 1, die an ihrer distalen Seite mit einem Paar von Sperrmanschetten (14) versehen ist, deren jede aus einer flüssigkeitsundurchlässigen Lage (26) gebildet ist und eine obere Seitenkante mit elastischer Dehnbarkeit in der Längsrichtung sowie eine mit der oberen Fläche der Windel einstückige proximale Seitenkante (28) aufweist, so daß die distale Seitenkante (29) bei Längswölbung der Windel von deren oberer Fläche aufklappen kann.

3. Windel nach Anspruch 2, wobei die proximale Seitenkante (28) mindestens in dem in Längsrichtung mittigen Bereich der Dichtmanschette zwischen der zugehörigen Seitenkante (22) des Kerns (4) und dem elastischen Teil (18) liegt und wobei die Dichtmanschette zwischen dem proximalen Seitenabschnitt (28) und dem elastischen Teil (18) in die Zonen hoher und geringer Steifigkeit unterteilt ist.

4. Windel nach Anspruch 1, wobei die Dichtmanschette mit einem einzelnen oder mehreren parallelen elastischen Teilen (18) versehen ist und ein innerhalb des innersten elastischen Teils verlaufender Abschnitt der Dichtmanschette in die Zonen hoher und geringer Steifigkeit unterteilt ist.

5. Windel nach Anspruch 1, wobei die Zone (42) hoher Steifigkeit die obere und/oder die untere Lage sowie eine von oberen und/oder der unteren Lage separat hergestellte und mit einer dieser Lagen verbundene Lage (26) umfaßt.

6. Windel nach Anspruch 1, wobei der Abstand von dem innersten elastischen Teil des elastischen Beinlochteils zu der Zone hoher Steifigkeit im Bereich von 1 bis 10 mm liegt.

7. Windel nach einem der vorhergehenden Ansprüche, wobei die Zone geringer Steifigkeit eine Querabmessung zwischen 5 und 100 mm hat.

8. Windel nach einem der vorhergehenden Ansprüche, wobei der Abstand zwischen einer Kante des absorbierenden Kerns und der Zone hoher Steifigkeit zwischen 0 und 30 mm liegt.

## Revendications

1. Couche-culotte jetable (1) présentant une direction transversale s'étendant circonférentiellement autour du torse d'un porteur et une direction longitudinale orthogonale à ladite direction transversale, comprenant une feuille supérieure perméable aux liquides (2), une feuille inférieure imperméable aux liquides (3), une partie centrale absorbant les liquides (4) disposée entre celles-ci et une paire de parements d'étanchéité (13) se situant à l'extérieur des bords latéraux transversalement opposés de ladite partie centrale s'étendant dans ladite direction longitudinale et présentant une extensibilité élastique dans ladite direction longitudinale, chaque dit parement d'étanchéité étant formé au moins partiellement par ladite feuille inférieure imperméable aux liquides (3) s'étendant latéralement depuis lesdits bords latéraux de ladite partie centrale,
et au moins une feuille séparée (26) stratifiée avec ladite feuille imperméable aux liquides et dotée de ladite extensibilité élastique en fixant un élément élastique de trou pour jambe (18) s'étendant dans la direction longitudinale, audit parement d'étanchéité (13) le long d'une ligne écartée du bord latéral associé de ladite partie centrale,
**caractérisée en ce que** ladite feuille supérieure (2) s'étend dans ladite direction transversale, au moins dans une zone intermédiaire longitudinale de ladite couche culotte, dans une partie (42) dudit parement d'étanchéité définie entre ledit bord latéral (23) de ladite partie centrale (4) et ledit élément élastique, le bord de ladite feuille supérieure redéfinissant une limite séparant le parement d'étanchéité, au moins dans une zone intermédiaire longitudinale de ladite couche-culotte, en une zone à rigidité élevée (42) et une zone à rigidité faible (41) de sorte que ladite zone à rigidité élevée (42), constituant ladite feuille supérieure (2), se situe de façon adjacente à ladite partie centrale, et que ladite zone à rigidité faible se situe de façon adjacente audit élément élastique, où ladite zone à rigidité élevée, constituant ladite feuille supérieure, présente une dimension transversale comprise entre 10 et 50 mm.

2. Couche-culotte selon la revendication 1, dans laquelle ladite couche-culotte (1) est munie sur son côté distal d'une paire de parements antifuites (14) formés chacun d'une feuille imperméable aux liquides (26) et comportant un bord latéral supérieur ayant une extensibilité élastique dans ladite direction longitudinale et un bord latéral proximal (28) solidaire de ladite surface supérieure de ladite couche-culotte, de sorte que ledit bord latéral distal (29) peut remonter à partir de ladite surface supérieure de ladite couche-culotte lorsque ladite couche-culotte est courbée longitudinalement.

3. Couche-culotte selon la revendication 2, dans laquelle ledit bord latéral proximal (28) se situe entre le bord latéral associé (22) de la partie centrale (4) et ledit élément élastique (18), au moins dans ladite zone intermédiaire longitudinale dudit parement d'étanchéité, et dans laquelle ledit parement d'étanchéité est divisé, entre ladite partie de bord latéral proximal (28) et ledit élément élastique (18), en ladite zone à rigidité élevée et ladite zone à rigidité faible.

4. Couche-culotte selon la revendication 1, dans laquelle ledit parement d'étanchéité est doté d'un seul ou de plusieurs éléments élastiques parallèles (18), et une partie dudit parement d'étanchéité s'étendant à l'intérieur de l'élément élastique le plus à l'intérieur est divisée en ladite zone à rigidité élevée et ladite zone à rigidité faible.

5. Couche-culotte selon la revendication 1, dans laquelle ladite zone à rigidité élevée (42) comprend au moins l'une desdites feuilles supérieure et inférieure, ainsi qu'une feuille (26) préparée séparément de ladite feuille supérieure et/ou de ladite feuille inférieure et collée à l'une desdites feuilles supérieure et inférieure

6. Couche-culotte selon la revendication 1, dans laquelle la distance de l'élément élastique le plus à l'intérieur dudit élément élastique de trou pour jambe à la zone à rigidité élevée, se situe dans une plage de 1 à 10 mm.

7. Couche-culotte selon l'une quelconque des revendications précédentes, dans laquelle ladite zone à rigidité faible présente une dimension transversale entre 5 et 100 mm.

8. Couche-culotte selon l'une quelconque des revendications précédentes, dans laquelle la distance entre un bord de ladite partie centrale absorbante et la zone à rigidité élevée se situe entre 0 et 30 mm.
